# EUROPEAN PATENT SPECIFICATION

(11) **EP 3 413 038 B1**
(45) Date of publication and mention of the grant of the patent: **17.03.2021**
(21) Application number: 18175053.0
(22) Date of filing: 30.05.2018
(51) Int. Cl.: G01N 23/223, G01N 33/38

(54) **PRESSED POWDER SAMPLE MEASUREMENTS USING X-RAY FLUORESCENCE**
MESSUNG GEPRESSTER PULVERPROBEN MITTELS RÖNTGENFLUORESZENZ
MESURES D'ÉCHANTILLONS DE POUDRE COMPACTÉE UTILISANT LA FLUORESCENCE DE RAYONS X

(30) Priority: 30.05.2017 US 201715608533
(43) Date of publication of application: 12.12.2018
(73) Proprietor: Malvern Panalytical B.V., 7602 EA Almelo (NL)
(72) Inventor: VREBOS, Bruno Alfred Robert, 7602 EA Almelo (NL); KUIPER, Dick, 7602 EA Almelo (NL); BEERKENS, Saskia Maria Angela, 7602 EA Almelo (NL)
(74) Representative: Elkington and Fife LLP

(56) References cited:
- VAN DEN BERG B C J ET AL: "Astronomical tuning for the upper Messinian Spanish Atlantic margin: Disentangling basin evolution, climate cyclicity and MOW", GLOBAL AND PLANETARY CHANGE, ELSEVIER, AMSTERDAM, NL, vol. 135, 20 October 2015 (2015-10-20), pages 89-103, XP029331255, ISSN: 0921-8181, DOI: 10.1016/J.GLOPLACHA.2015.10.009
- C. VAN ZYL: "Rapid preparation of robust pressed powder briquettes containing a styrene and wax mixture as binder", X-RAY SPECTROMETRY, vol. 11, no. 1, January 1982 (1982-01), pages 29-31, XP055518549, GB ISSN: 0049-8246, DOI: 10.1002/xrs.1300110108
- N. K. SAINI ET AL: "Evaluation of energy-dispersive x-ray fluorescence spectrometry in the rapid analysis of silicate rocks using pressed powder pellets", X-RAY SPECTROMETRY, vol. 29, no. 2, March 2000 (2000-03), pages 166-172, XP055518668, GB ISSN: 0049-8246, DOI: 10.1002/(SICI)1097-4539(200003/04)29:2<166 ::AID-XRS411>3.0.CO;2-L

## Description

### Field of Invention

The invention relates to a method of X-ray fluorescence analysis of pressed powder samples.

### Background to the Invention

Quantitative X-ray fluorescence measurements may be made by measuring the intensity of X-ray fluorescence and calculating a concentration of a particular element in the sample based on the measured intensity.

In order to prepare samples for X-ray fluorescence measurements, for some samples a pellet is prepared by pressing the powder into a pellet. Powdered samples first may be milled to a fine powder. If the powder in the as-received state is fine enough, then often the milling step is not performed. The loose powder is then pressed into a pellet. In order to produce pellets that are strong enough to withstand the normal operating conditions (transportation, loading and unloading, vacuum conditions) it may be required to add a binding agent prior to pressing.

However, quantitative theories of X-ray analysis are based on the assumption that the sample is homogenous at the length scale of the path lengths of the X-rays involved - these path lengths are typically smaller than 10 µm. This assumption is never true when analysing powders, whether the powder is pressed or loose. This affects the accuracy of the measurement. For this reason, an alternative sample preparation by fusing (melting) a sample is sometimes adopted for quantitative X-ray fluorescence measurements. Fusion is however not practicable for all samples, and moreover is considerably more difficult, time consuming and expensive than using pressed powder samples.

In order to reduce negative effects from using a pressed powder sample, the risks can be significantly mitigated by using a fixed, well-documented and consistently executed sample preparation program for the samples to be measured and for reference samples of known composition. It is important to be consistent with procedure used as well as details of the mixing. Consistency in this preparation process means that any effects of non-ideality of the sample are the same for the measured samples and the reference materials, minimising the consequences of the non-ideality of the sample.

Of course, this means that the reference samples must be of the same type as the samples of interest and have the same physical properties, such as grain size, grain size distribution, phases present, and composition.

A problem can occur when suitable reference standards are not available. This is not a new problem and is a particular issue when quantitative analysis is performed with non-type standards. In particular, "standard-less" approaches may be used to convert measured intensity values into concentration - these are known by SQS, PSA, SSQ, UniQuant, IQ, Omnian, and others.

There is therefore a need for an improved approach to evaluating results on powder samples for quantitative analysis.

C. Van Zyl: "Rapid preparation of robust pressed powder briquettes containing a styrene and wax mixture as binder" , X-ray Spectrometry, vol. 11, no. 1, January 1982, pages 29-31 teaches a process for making sample briquettes.

N. K. Saini et al.: Evaluation of energy-dispersive x-ray fluorescence spectrometry in the rapid analysis of silicate rocks using presser powder pellets", X-Ray Spectrometry, vol. 29, no. 2, March 2000, pages 166-172 teaches a technique for analysing silicate rocks using pressed power pellets.

B.C.J. van den Berg et al.: "Astronomical tuning for the upper Messinian Spanish Atlantic margin: Disentangling basin evolution, climate cyclicity and MOW", Global and Planetary Change, vol. 135, 20 October 2015 (2015-10-20), pages 89-103, teaches a method of analysing pressed powder samples having a binding agent by quantitative X-ray fluorescence spectroscopy. Chemical matrix effects are being corrected for by the software used.

### Summary of the invention

According to a first aspect of the invention there is provided a method according to claim 1.

The inventors have realised that one reason for inaccurate results on pressed powder samples using a binder is that the binder may segregate and that this may be taken into account by describing the analyzed volume of the specimen as having a different physical representation than the specimen's bulk properties with respect to the relative amounts of binder and original powder sample. The final representation of the specimen's analyzed volume then involves an iterative procedure, including the amount of the binder added.

Note that, unexpectedly, it is not required to constrain the physical representation of the specimen for the analysis to correspond to the sample with binder itself. For example, as discussed below, the calculations may assume a surface layer of pure or nearly pure binder which may not be present in the actual sample. Alternatively, the calculations may assume a homogenous mixture of binder and sample material having a concentration of binder very different to the bulk concentration of binder.

In one embodiment, the quantity of binder assumed is the concentration of binder in the sample, which may be expressed as percentage (0 to 100%) or as a fraction (0 to 1).

In another embodiment, the pressed sample is modelled as a sample having a thin layer of binder on the surface. The layer may be a layer of pure binder. In this case, the quantity of binder assumed is the thickness of an assumed layer of pure binder on the surface of the pressed sample.

The inventors have realised that by modelling the segregation as a thin layer of binder at the surface of the sample together with a homogenous middle region of the sample a good approximation to measured data may be obtained which still allows for mathematical calculation.

In another embodiment, the pressed specimen is modelled as consisting of the bulk material and a thin layer of a mixture of binder and sample on the surface. This is equivalent to considering the pellet as having a thin layer that is highly enriched in binder on the analytical surface. This could be caused by factors such as different flow properties, different grain sizes, and differences in compressibility of binder and the powdered sample.

In this example the specimen as presented to the X-ray spectrometer is described as consisting of a bulk specimen (sample + binder) coated with a thin layer of different composition - binder only or another mix of binder and sample. Frequently, the thin layer may be considered to consist of (nearly) pure binder. The thickness of the layer is determined in an iterative way. Starting from an initial value for the thickness of the layer of binder, the total of the concentrations is calculated. Based on the value of that total of the concentrations thus determined, the thickness of the layer is changed. The process stops when the total of concentrations sum up to 100% (if the composition is calculated in percentages w/w) or unity (if the calculations are done on mass fractions);

The method also relates to a quantitative X-ray analysis method, comprising:
forming a sample into a pellet using a binder
carrying out an X-ray fluorescence measurement; and
evaluating the results using a method as set out above.

### Brief description of the drawings

Embodiments of the invention will now be described with reference to the accompanying diagrams, in which:
Figure 1 shows a pressed powder sample with a uniform mixture of powder and binder;
Figure 2 shows a sample with segregation;
Figure 3 shows a model sample used in an embodiment of the invention;
Figure 4 shows a calibration graph of a comparative example;
Figure 5 shows a calibration graph of a method according to an embodiment of the invention;
Figure 6 shows a flow chart of a method according to an embodiment of the invention; and
Figure 7 shows apparatus in accordance with an embodiment of the invention.

### Detailed description

Embodiments of the invention will now be presented with reference to examples.

Many of the examples use the same sample, a sample with the concentrations of the following oxides (expressed in %).

| | | | | | |
|---|---|---|---|---|---|
| Na₂O | MgO | Al₂O₃ | SiO₂ | CaO | Fe₂O₃ |
| 0.281 | 2.079 | 4.716 | 20.225 | 63.992 | 3.472 |

These are measured values from a pressed powder sample without any binder - the measured values sum to 99.113%. The discrepancy with 100% may be caused by sample preparation as discussed above as well as the presence of other elements in low concentration. In order to form a pellet, the pressure needed was 200 kN. The sample is a reference sample that is very carefully prepared for homogeneity. The measured results are presented in the first line of the following table, labeled "No binder".

**Table 1**

| Sample | Sum | Na₂O | MgO | Al₂O₃ | SiO₂ | CaO | Fe₂O₃ |
|---|---|---|---|---|---|---|---|
| No Binder | 99.113 | 0.281 | 2.079 | 4.716 | 20.225 | 63.992 | 3.472 |
| 5% 100 kN Licowax | 94.289 | 0.250 | 1.810 | 4.194 | 18.484 | 62.043 | 3.456 |
| Difference | | 0.031 | 0.269 | 0.522 | 1.741 | 1.949 | 0.016 |
| X1.061 | 100.000 | 0.265 | 1.920 | 4.448 | 19.604 | 65.801 | 3.665 |
| Difference | | 0.016 | 0.159 | 0.268 | 0.621 | 1.809 | 0.193 |
| **25% input** | 99.996 | 0.282 | 2.032 | 4.677 | 20.434 | 64.763 | 3.470 |
| Difference with 0% binder | | 0.001 | 0.047 | 0.039 | 0.209 | 0.771 | 0.002 |

The same powder was then pressed into a specimen using 5% Licowax and a pressure of 100kN. The specimen was measured and the results shown in the row of the table above labelled "5% 100 kN Licowax". The sum of the measured concentrations, excluding binder, adds up to 94.289%. If it is assumed that the concentrations should add to 100%, this means that each concentration should be multiplied by 1.061 to arrive at the concentration in the original sample, without the binder. The results are presented in the line "x1.061". This scaling typically improves the results of some analytes, while adversely affecting others. Accordingly, there are significant differences between these values and the actual percentages of each of the components, as indicated above.

To correct this, a method according to the invention was carried out. In this case, the amount of binder is not considered fixed but is instead considered to be a variable parameter. In an initialisation step, an initial percentage of binder is assumed (which may be zero but not negative) - this may be referred to as the assumed concentration of binder.

Those skilled in the art will realise that in order to accurately calculate the quantities of the components from the measured X-ray intensities, it is necessary to take into account the amounts of all components, including the binder. For example, the X-rays passing through the sample, both before and after X-ray fluorescence, may be absorbed both by the binder and each of the components of the specimen. The calculations of the quantities of each of the components accordingly take into account the amount of binder, and preferably the other components, for example the absorbance of X-rays by the binder as the X-rays pass through the sample. Failure to accurately account for these effects would result in poor, non-quantitative results.

Note that it is not normally possible to directly measure the concentration of the binder, but this is calculated from the quantity of other components from the measured intensities of those components and then assuming that the remainder is binder.

Accordingly, taking the assumed percentage of binder into account the composition of the specimen is calculated. The total of the concentrations of the components other than the binder is also calculated.

Based on the value of that total, the amount of binder is recalculated, and the assumed concentration of binder changed to the recalculated value. The composition of the specimen is calculated again. The process stops when the total of concentrations sum up to approximately 100% (if the composition is calculated in percentages w/w) or unity (if the calculations are done on mass fractions).

Note that in practice the convergence criteria may be to stop when one of the following occurs:
(a) after the sum of concentrations is 100% plus or minus a predetermined range, for example within a deviation from 100% between 0.001% and 0.01% depending on the accuracy required;
(b) after the change between successive iterations is smaller than a predetermined change, for example 0.0001 %; or
(c) after a predetermined number of iterations.

By including criterion (c) the iteration is stopped even if convergence does not occur and ensure that the computer program is correctly terminated in this eventuality.

This method may be understood more readily with reference to an example. Consider the case where as a starting point the assumed value is 5% of binder. The total concentration of components is then calculated, including the binder. In an example, the total concentration of components sums to 93%.

Then, in this first iteration, there is (100%-93%)=7% missing, the assumed concentration of binder is raised by 7%, to 12% for a second iteration.

In the second iteration the total concentration of each of their components is then calculated again. In this example, say the total concentration is now 101%. In this case, the total amount of binder is adjusted to 11%, to adjust the assumed binder concentration to 100% for the third iteration.

In the third iteration, the concentration of each component is calculated again. Assume in this case the total concentration comes to 99.8%. The binder concentration is now adjusted upwards by 0.2% so the assumed binder concentration for a fourth iteration is 11.2%.

In the fourth iteration, the concentration of each component is calculated again. Assume in this case that the total is 100.002%. This is close enough to 100% for the calculation to stop. The concentrations are then output.

It will be understood by those skilled in the art that there are a variety of approaches to carrying out such iterative processes. In particular, it is not necessary to change the concentration of binder by exactly the amount of the fraction missing by subtraction - other approaches may calculate a fractional increase (i.e. a percentage change in the percentage of binder).

Returning to the specific example in table 1 above, this method was carried out and converged to 100% plus or minus a predetermined amount with surprisingly high percentage of binder of 25%. However, the method is not designed to obtain the percentage of binder, but the relative percentages of the components of the original specimen without binder. Reviewing the results in table 1, it is apparent that the results with this assumed percentage of binder of 25% give better results for the relative composition of the original specimen. Comparing these results to the concentration obtained on a specimen without binder (first line in the table), it is clear that the results are now in very good agreement.

In other words, in this example good results can be obtained from the measurements made using a binder with a percentage of binder of 25%. Note that this is a very different percentage to the actual percentage of 5%.

This approach was carried out for a number of samples, and further results will now be presented in more detail in table 2. The data is for a single material, prepared in different ways. The homogeneity with respect to composition of the bulk material has been verified by taking different samples and analyzing these on high-end WDXRF equipment. No statistical evidence of homogeneity issues regarding composition of the bulk material has been found. The effects described are thus not attributable to differences in composition of the individual specimens rested. Furthermore, all samples have been made in triplicate. Based on statistical analysis, the reproducibility of the specimen preparation is such that it does not contribute to the effect (i.e. the differences in concentrations) observed.

The data in the table has been generated using a PANalytical E3 XRF apparatus with Omnian software.

The bulk material was prepared into samples for X-ray fluorescence measurements with three different binders, in each case with 5%, 10% and 25% binder, leading to nine different results. The first example is the same as presented above, and the results are presented in the same way. As above, the first line of results is the direct true values results, the third line the results corrected so that the results sum to 100%, the fourth line the difference between that and the true values, the fifth line the results calculated using an unrealistic amount of binder, and the sixth line the difference between those results and the true values.

Note that in all cases the results of the final calculation, in accordance with the invention, are closest to the reference values.

Note however that the samples with higher real percentages of binder also have higher assumed percentages of binder.

As will be apparent, the percentage of binder that results from the method is much higher than the actual percentage of binder in the sample. However, this does not matter since the point of the experiment is to measure the percentage of different materials in the original sample, and not to measure the binder. The inventors have realised that carrying out the calculations with such unrealistic values of the binder give rise to improved results.

This method can be viewed as dealing with pressed pellet specimens where the binder and sample are not distributed uniformly. This can be caused by the different properties of the powdered sample and the binder material when compressed.

**Table 2**

| Sample | Sum | Na₂O | MgO | Al₂O₃ | SiO₂ | CaO | Fe₂O₃ |
|---|---|---|---|---|---|---|---|
| No Binder | 99.113 | 0.281 | 2.079 | 4.716 | 20.225 | 63.992 | 3.472 |
| 5% 100 kN Licowax | 94.289 | 0.250 | 1.810 | 4.194 | 18.484 | 62.043 | 3.456 |
| Difference | | 0.031 | 0.269 | 0.522 | 1.741 | 1.949 | 0.016 |
| X1.061 | 100.000 | 0.265 | 1.920 | 4.448 | 19.604 | 65.801 | 3.665 |
| Difference | | 0.016 | 0.159 | 0.268 | 0.621 | 1.809 | 0.193 |
| **25% binder** | 99.996 | 0.282 | 2.032 | 4.677 | 20.434 | 64.763 | 3.470 |
| Difference with 0% binder | | 0.001 | 0.047 | 0.039 | 0.209 | 0.771 | 0.002 |
| 10% 100 kN Licowax | 92.422 | 0.231 | 1.712 | 3.988 | 17.716 | 61.342 | 3.465 |
| Difference | | 0.050 | 0.367 | 0.728 | 2.509 | 2.650 | 0.007 |
| X1.082 | 99.998 | 0.269 | 1.985 | 4.588 | 20.173 | 65.132 | 3.507 |
| Difference | | 0.012 | 0.094 | 0.128 | 0.052 | 1.140 | 0.035 |
| **33% binder** | 99.847 | 0.270 | 1.990 | 4.596 | 20.199 | 64.962 | 3.488 |
| Difference with 0% binder | | 0.011 | 0.089 | 0.120 | 0.026 | 0.970 | 0.016 |
| 25% 100 kN Licowax | 84.299 | 0.168 | 1.319 | 3.302 | 14.896 | 57.640 | 3.415 |
| Difference | | 0.113 | 0.760 | 1.414 | 5.329 | 6.352 | 0.057 |
| X1.189 | 100.001 | 0.199 | 1.565 | 3.917 | 17.671 | 68.374 | 4.051 |
| Difference | | 0.082 | 0.514 | 0.799 | 2.554 | 4.382 | 0.579 |
| **58% binder** | 99.658 | 0.232 | 1.799 | 4.440 | 19.646 | 65.723 | 3.496 |
| Difference with 0% binder | | 0.049 | 0.280 | 0.276 | 0.579 | 1.731 | 0.024 |
| 5% 100 kN Ultrawax | 95.272 | 0.254 | 1.838 | 4.297 | 18.787 | 62.479 | 3.469 |
| Difference | | 0.027 | 0.241 | 0.419 | 1.438 | 1.513 | 0.003 |
| X1.050 | 99.999 | 0.267 | 1.929 | 4.510 | 19.719 | 65.579 | 3.642 |
| Difference | | 0.014 | 0.150 | 0.206 | 0.506 | 1.587 | 0.170 |
| **25% binder** | 99.900 | 0.281 | 2.022 | 4.696 | 20.382 | 64.655 | 3.480 |
| Difference with 0% binder | | 0.000 | 0.057 | 0.020 | 0.157 | 0.663 | 0.008 |
| 5% 100 kN Ultrawax | 92.391 | 0.232 | 1.722 | 4.043 | 17.812 | 61.101 | 3.449 |
| Difference | | 0.049 | 0.357 | 0.673 | 2.413 | 2.891 | 0.023 |
| X1.082 | 99.997 | 0.251 | 1.864 | 4.376 | 19.279 | 66.113 | 3.733 |
| Difference | | 0.030 | 0.215 | 0.340 | 0.946 | 2.121 | 0.261 |
| **37% binder** | 99.847 | 0.273 | 2.007 | 4.668 | 20.332 | 64.683 | 3.469 |
| Difference with 0% binder | | 0.008 | 0.072 | 0.048 | 0.107 | 0.691 | 0.003 |
| 25% 100 kN Ultrawax | 85.862 | 0.188 | 1.422 | 3.505 | 15.650 | 57.980 | 3.396 |
| Difference | | 0.093 | 0.657 | 1.211 | 4.575 | 6.012 | 0.076 |
| X1.165 | 100.003 | 0.218 | 1.657 | 4.082 | 18.226 | 67.526 | 3.955 |
| Difference | | 0.063 | 0.422 | 0.634 | 1.999 | 3.534 | 0.483 |
| **60% binder** | 100.367 | 0.256 | 1.910 | 4.627 | 20.263 | 65.386 | 3.463 |
| Difference with 0% binder | | 0.025 | 0.169 | 0.089 | 0.038 | 1.394 | 0.009 |
| 5% 100 kN Cellulose | 93.788 | 0.253 | 1.849 | 4.252 | 18.684 | 61.365 | 3.419 |
| Difference | | 0.028 | 0.230 | 0.464 | 1.541 | 2.627 | 0.053 |
| X1.066 | 99.996 | 0.269 | 1.972 | 4.533 | 19.921 | 65.429 | 3.645 |
| Difference | | 0.012 | 0.107 | 0.183 | 0.304 | 1.437 | 0.173 |
| **20% binder** | 99.729 | 0.285 | 2.076 | 4.747 | 20.713 | 64.212 | 3.437 |
| Difference with 0% binder | | 0.004 | 0.003 | 0.031 | 0.488 | 0.220 | 0.035 |
| 10% 100 kN Cellulose | 92.303 | 0.244 | 1.845 | 4.225 | 18.362 | 60.298 | 3.405 |
| Difference | | 0.037 | 0.234 | 0.491 | 1.863 | 3.694 | 0.067 |
| X1.083 | 100.000 | 0.265 | 1.999 | 4.577 | 19.893 | 65.326 | 3.689 |
| Difference | | 0.016 | 0.080 | 0.139 | 0.332 | 1.334 | 0.217 |
| **28% binder** | 100.330 | 0.286 | 2.153 | 4.894 | 21.072 | 64.175 | 3.433 |
| Difference with 0% binder | | 0.005 | 0.074 | 0.178 | 0.847 | 0.183 | 0.039 |
| 25% 100 kN Cellulose | 86.454 | 0.231 | 1.693 | 3.894 | 16.812 | 56.840 | 3.313 |
| Difference | | 0.050 | 0.386 | 0.822 | 3.413 | 7.152 | 0.159 |
| X1.157 | 100.020 | 0.267 | 1.959 | 4.504 | 19.447 | 65.747 | 3.832 |
| Difference | | 0.014 | 0.120 | 0.212 | 0.778 | 1.755 | 0.360 |
| **47% binder** | 100.167 | 0.300 | 2.189 | 4.985 | 21.232 | 63.741 | 3.386 |
| Difference with 0% binder | | 0.019 | 0.110 | 0.269 | 1.007 | 0.251 | 0.086 |

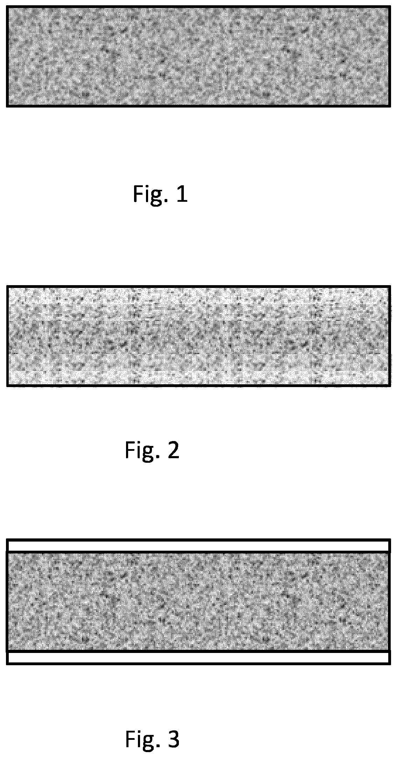

Without wishing to be bound by theory, the inventors believe that the calculation works with percentages of binder widely different from the bulk composition (which can be calculated based on the masses of the powder sample and the binder added) due to the fact that the analyzed volume (which is very small) has a composition different from that of the bulk. In other words, the method takes account of the fact that the binder is not homogeneously distributed throughout the sample. Figures 1, 2 and 3 illustrate this.

Figure 1 illustrates the theoretical state in which the binder is uniformly distributed throughout the sample used for X-ray measurements. In this case, the concentrations of the analyzed volume and the bulk are the same.

In practice, some segregation will occur and the binder will be present in higher quantities near the surface, as illustrated in Figure 2 - note that the binder is in this drawing presented as white.

The effects of this segregation can be corrected using the method as described above of treating the percentage of binder as a variable.

As an alternative way of dealing with the segregation of the binder, it is possible to assume that the sample consists of two thin layers of pure binder sandwiching the rest of the sample, as illustrated in Figure 3. In this case, the amount of binder is varied not by varying the percentage of binder, but instead by varying the thickness of the surface layer of binder and again carrying out an iterative process. Note that the model is not necessarily an accurate representation of reality in that the actual arrangement of binder is more similar to that indicated in Figure 2 and not that indicated in Figure 3, but the inventors have realised that the use of this model - though not accurately representing the real situation - does allow for more accurate measurement of the real composition of the sample.

Without wishing to be bound by theory, it is believed that the effect of the gradual change in composition (binder relative to sample) with distance within the specimen may be replaced in this method with a thin layer of a given composition (binder or binder + sample) that has the same absorption properties as the gradual change.

A value of the thickness of the binder in the model is assumed, calculations are carried out based on the captured data, and the total concentration calculated. Based on that calculation, the assumed thickness of the layer of binder is changed and the total concentration calculated again. The process is repeated until a value for the total concentration of all components of the original sample sums to 100% if the concentration is expressed as a percentage or unity if the concentration is expressed as a fraction.

The method of Figure 3 may be varied by assuming that the layers on the surface are not pure binder but a layer enriched in binder, i.e. a layer in which the percentage of binder is much higher than the bulk percentage in the sample as a whole. Again, this representation does not match the physical reality.

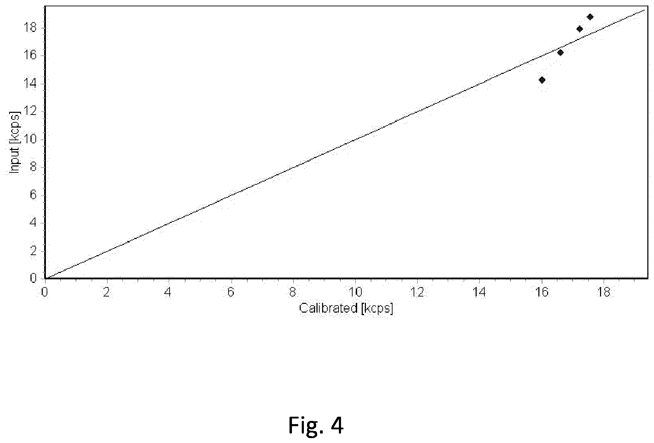

Figure 4 illustrates a calibration curve (as a comparative example) obtained for the same sample as above with variable amounts of wax, using as a measurement line the Mg K alpha 1,2 line. In the case of Figure 4, the wax is assumed simply to dilute the sample. The different points on the graph relate to different amounts of wax. Each point has the measured value of the count on the vertical axis and the calculated count on the horizontal.

If the wax did simply act as a diluent the points should form a straight line passing through the origin, as indicated by the line on the graph. However, it is apparent that the points do not all on this straight line passing through the origin. The assumption of homogeneity is accordingly not correct.

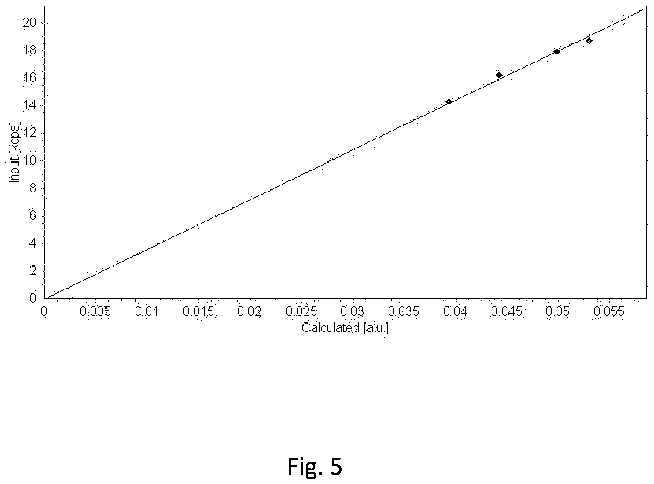

Referring to Figure 5, the same data is plotted using the method described above in which a thin layer of pure binder is assumed to be present. This is a significant improvement on Figure 4.

This graph shows that using the assumption of a thin layer of binder much better results can be obtained.

In general terms, the two methods described above both treat the amount of binder as a variable. The amount of binder is assumed - either a percentage, fraction, or the thickness of a thin layer of binder - and the concentrations of the various components of the sample calculated. The amount of binder is varied and the calculations repeated until the concentrations sum to the correct value. In the case where all components of a sample are measured, that will be 100% or unity in the case of a fractional representation.

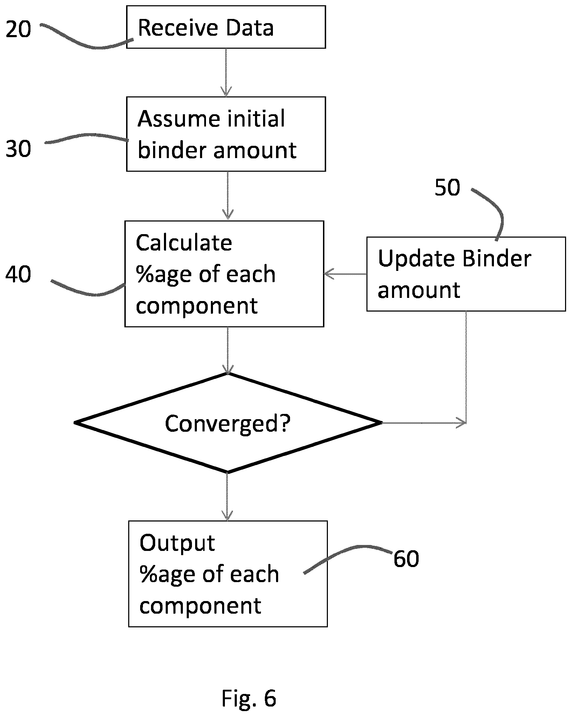

The method may be expressed as a flow chart as illustrated in Figure 6.

The measured XRF data of a sample with a plurality of components is entered into the system in step 20. Next, a quantity of binder is assumed- either percentage or thickness of a thin layer of pure binder at the surface in step 30.

Next, the percentage of each component is calculated in step 40. The percentage of binder is updated in step 50, and these steps repeated until the calculations converge.

After the calculations converge, the measured concentrations are output in step 60.

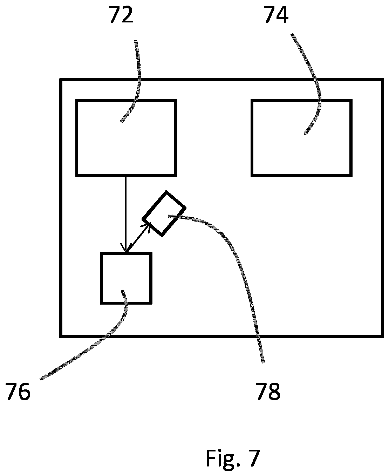

Apparatus for carrying out the invention is illustrated schematically in Figure 7. An X-ray source 72 directs X-rays at a sample 76 which emits X-rays as a result of X-ray fluorescence. The X-rays are captured by X-ray detector 78 and the data is passed to a computer 74 which in the drawing is shown as part of the X-ray apparatus but which may also be a stand-alone computer. The computer contains software code for automatically carrying out examples of the method as set out above. The computer may also contain software code for controlling the X-ray source, X-ray detector, and other components of the X-ray fluorescence apparatus, as will be understood by the skilled person.

The skilled person will understand that the apparatus discussed above is schematic and will be aware of other components and arrangements that will allow the use of the method. For example, for wavelength dispersive XRF instrumentation as well as some more advanced XRF instrumentation, there may be additional components located between position 72 and position 76 such as secondary targets, diffracting crystals (e.g. highly oriented pyrolytic graphite (HOPG)), or primary beam filters. Similarly, there might be for example a monochromatising crystal between positions 76 and 78. The method applies to all these configurations.

The results presented above show that in these examples the assumed amount of binder for the calculations to converge is higher than the actual amount of binder, which is assumed to be because the binder segregates to the surface. However, the method can also be used for a binder which is present in smaller quantities at the surface than in the bulk.

## Claims

1. A quantitative X-ray analysis method, comprising:
receiving X-ray fluorescence data (20) of a pressed sample (76) prepared using an additional binder;
initialising an assumed quantity of binder (30);
calculating the concentration of a plurality of components of the sample (40) based on the X-ray fluorescence data and the assumed quantity of binder;
changing the assumed quantity of binder (50) based on the results of the step of calculating the concentration of a plurality of components;
repeating the step of calculating the concentration of a plurality of components of the sample and the step of changing the assumed quantity of binder until the calculations converge; and
outputting the concentration of components of the sample (60).

2. A method according to claim 1, wherein the changed assumed concentration of binder is calculated by summing the calculated concentration of the said plurality of components and assuming that the remainder is binder.

3. A method according to claim 1, wherein
the quantity of binder assumed is the concentration of binder in the sample.

4. A method according to claim 3, wherein calculating the concentration of a plurality of components includes taking into account absorbance by the binder and absorbance by the plurality of components.

5. A method according to claim 1 wherein
the quantity of binder assumed is the thickness of an assumed layer of pure binder on the surface of the pressed sample.

6. A method according to claim 1, wherein the quantity of binder assumed includes the thickness of an assumed layer on the surface of the pressed sample and the concentration of binder in the assumed layer.

7. A method according to claim 1, wherein the quantity of binder assumed includes a first quantity of binder, being the thickness of an assumed layer of binder on the surface of the pressed sample, and the second quantity of binder being the concentration of binder in the bulk in the sample.

8. A quantitative X-ray analysis method, comprising:
forming a sample into a pellet using a binder
carrying out an X-ray fluorescence measurement; and
evaluating the results using a method according to any preceding claim.

9. Apparatus for quantitative X-ray analysis of a pressed powder sample (76) containing a binder, comprising:
a computer (74) containing code means adapted to cause the computer to carry out a method on X-ray fluorescence data from the pressed powder sample, the method comprising:
receiving X-ray fluorescence data of a pressed sample prepared using an additional binder;
initialising an assumed quantity of binder;
calculating the concentration of a plurality of components of the sample based on the X-ray fluorescence data and the assumed quantity of binder;
changing the assumed quantity of binder based on the results of the step of calculating the concentration of a plurality of components;
repeating the step of calculating the concentration of a plurality of components of the sample and the step of changing the assumed quantity of binder until the calculations converge; and
outputting the concentration of components of the sample.

10. Apparatus for quantitative X-ray analysis of a pressed powder sample containing a binder according to claim 9, further comprising:
an X-ray source (72) for generating X-rays and directing them to a sample (76); and
an X-ray detector for capturing X-ray fluorescence generated by the sample;
wherein the X-ray source and X-ray detector are connected to and controlled by the computer (74).

## Patentansprüche

1. Quantitatives Röntgenanalyseverfahren, umfassend:
Empfangen von Röntgenfluoreszenzdaten (20) einer gepressten Probe (76), die unter Verwendung eines zusätzlichen Bindemittels hergestellt wird;
Initialisieren einer angenommenen Menge an Bindemittel (30);
Berechnen der Konzentration einer Vielzahl von Komponenten der Probe (40) basierend auf den Röntgenfluoreszenzdaten und der angenommenen Menge an Bindemittel;
Ändern der angenommenen Menge an Bindemittel (50) basierend auf den Ergebnissen des Schrittes des Berechnens der Konzentration einer Vielzahl von Komponenten;
Wiederholen des Schrittes des Berechnens der Konzentration einer Vielzahl von Komponenten der Probe und des Schrittes des Änderns der angenommenen Menge an Bindemittel, bis die Berechnungen konvergieren; und
Ausgeben der Konzentration an Komponenten der Probe (60).

2. Verfahren nach Anspruch 1, wobei die geänderte angenommene Konzentration an Bindemittel berechnet wird, indem die berechnete Konzentration der Vielzahl von Komponenten summiert wird und angenommen wird, dass der Rest Bindemittel ist.

3. Verfahren nach Anspruch 1, wobei
die angenommene Menge an Bindemittel die Konzentration an Bindemittel in der Probe ist.

4. Verfahren nach Anspruch 3, wobei das Berechnen der Konzentration einer Vielzahl von Komponenten das Berücksichtigen von Absorption durch das Bindemittel und von Absorption durch die Vielzahl von Komponenten beinhaltet.

5. Verfahren nach Anspruch 1, wobei
die angenommene Menge an Bindemittel die Dicke einer angenommenen Schicht aus reinem Bindemittel auf der Oberfläche der gepressten Probe ist.

6. Verfahren nach Anspruch 1, wobei die angenommene Menge an Bindemittel die Dicke einer angenommenen Schicht auf der Oberfläche der gepressten Probe und die Konzentration an Bindemittel in der angenommenen Schicht beinhaltet.

7. Verfahren nach Anspruch 1, wobei die angenommene Menge an Bindemittel eine erste Menge an Bindemittel beinhaltet, welche die Dicke einer angenommenen Schicht an Bindemittel auf der Oberfläche der gepressten Probe ist, und wobei die zweite Menge an Bindemittel die Konzentration an Bindemittel in der Masse in der Probe ist.

8. Quantitatives Röntgenanalyseverfahren, umfassend:
Bilden einer Probe zu einem Pellet unter Verwendung eines Bindemittels;
Durchführen einer Röntgenfluoreszenzmessung; und
Bewerten der Ergebnisse unter Anwendung eines Verfahrens nach einem vorhergehenden Anspruch.

9. Vorrichtung zur quantitativen Röntgenanalyse einer gepressten Pulverprobe (76), die ein Bindemittel enthält, umfassend:
einen Computer (74), der Codemittel enthält, die ausgelegt sind, um den Computer dazu zu veranlassen, ein Verfahren an Röntgenfluoreszenzdaten aus der gepressten Pulverprobe durchzuführen, wobei das Verfahren Folgendes umfasst:
Empfangen von Röntgenfluoreszenzdaten einer gepressten Probe, die unter Verwendung eines zusätzlichen Bindemittels hergestellt wird;
Initialisieren einer angenommenen Menge an Bindemittel;
Berechnen der Konzentration einer Vielzahl von Komponenten der Probe basierend auf den Röntgenfluoreszenzdaten und der angenommenen Menge an Bindemittel;
Ändern der angenommenen Menge an Bindemittel basierend auf den Ergebnissen des Schrittes des Berechnens der Konzentration einer Vielzahl von Komponenten;
Wiederholen des Schrittes des Berechnens der Konzentration einer Vielzahl von Komponenten der Probe und des Schrittes des Änderns der angenommenen Menge an Bindemittel, bis die Berechnungen konvergieren; und
Ausgeben der Konzentration an Komponenten der Probe.

10. Vorrichtung zur quantitativen Röntgenanalyse einer gepressten Pulverprobe, die ein Bindemittel enthält, nach Anspruch 9, ferner umfassend:
eine Röntgenquelle (72) zum Erzeugen von Röntgenstrahlen und zum Richten dieser auf eine Probe (76); und
einen Röntgendetektor zum Erfassen der Röntgenfluoreszenz, die durch die Probe erzeugt wird;
wobei die Röntgenquelle und der Röntgendetektor mit dem Computer (74) verbunden sind und durch diesen gesteuert werden.

## Revendications

1. Procédé d'analyse quantitative aux rayons X, comprenant :
la réception de données de fluorescence de rayons X (20) d'un échantillon compacté (76) préparé à l'aide d'un liant supplémentaire ;
l'initialisation d'une quantité supposée de liant (30) ;
le calcul de la concentration d'une pluralité de composants de l'échantillon (40) sur la base des données de fluorescence de rayons X et de la quantité supposée de liant ;
la modification de la quantité supposée de liant (50) sur la base des résultats de l'étape de calcul de la concentration d'une pluralité de composants ;
la répétition de l'étape de calcul de la concentration d'une pluralité de composants de l'échantillon et de l'étape de modification de la quantité supposée de liant jusqu'à ce que les calculs convergent ; et
l'émission en sortie de la concentration de composants de l'échantillon (60).

2. Procédé selon la revendication 1, ladite concentration supposée modifiée de liant étant calculée en additionnant la concentration calculée de ladite pluralité de composants et en supposant que le reste est du liant.

3. Procédé selon la revendication 1, ladite quantité de liant supposée étant la concentration de liant dans l'échantillon.

4. Procédé selon la revendication 3, ledit calcul de la concentration d'une pluralité de composants comprenant la prise en compte de l'absorbance par le liant et de l'absorbance par la pluralité de composants.

5. Procédé selon la revendication 1, la quantité de liant supposée étant l'épaisseur d'une couche supposée de liant pur sur la surface de l'échantillon compacté.

6. Procédé selon la revendication 1, ladite quantité de liant supposée comprenant l'épaisseur d'une couche supposée sur la surface de l'échantillon compacté et la concentration de liant dans la couche supposée.

7. Procédé selon la revendication 1, ladite quantité de liant supposée comprenant une première quantité de liant, qui est l'épaisseur d'une couche supposée de liant sur la surface de l'échantillon compacté, et la seconde quantité de liant qui est la concentration de liant en vrac dans l'échantillon.

8. Procédé d'analyse quantitative aux rayons X, comprenant : la formation d'un échantillon en une pastille à l'aide d'un liant, la réalisation d'une mesure de fluorescence de rayons X ; et
l'évaluation des résultats à l'aide d'un procédé selon l'une quelconque des revendications précédentes.

9. Appareil destiné à l'analyse quantitative aux rayons X d'un échantillon de poudre compacté (76) contenant un liant, comprenant :
un ordinateur (74) contenant un moyen de code adapté pour amener l'ordinateur à effectuer un procédé sur des données de fluorescence de rayons X à partir de l'échantillon de poudre compacté, le procédé comprenant :
la réception des données de fluorescence de rayons X d'un échantillon compacté préparé à l'aide d'un liant supplémentaire ;
l'initialisation d'une quantité supposée de liant ;
le calcul de la concentration d'une pluralité de composants de l'échantillon sur la base des données de fluorescence de rayons X et de la quantité supposée de liant ;
la modification de la quantité supposée de liant sur la base des résultats de l'étape de calcul de la concentration d'une pluralité de composants ;
la répétition de l'étape de calcul de la concentration d'une pluralité de composants de l'échantillon et de l'étape de modification de la quantité supposée de liant jusqu'à ce que les calculs convergent ; et
l'émission en sortie de la concentration de composants de l'échantillon.

10. Appareil destiné à l'analyse quantitative aux rayons X d'un échantillon de poudre compacté contenant un liant selon la revendication 9, comprenant en outre :
une source de rayons X (72) destinée à générer des rayons X et à les diriger vers un échantillon (76) ; et
un détecteur de rayons X destiné à capturer la fluorescence de rayons X générée par l'échantillon ; ladite source de rayons X et ledit détecteur de rayons X étant raccordés et commandés par l'ordinateur (74).
